(19) Europäisches Patentamt
European Patent Office
Office européen des brevets

(11) Publication number: **0 008 186 A1**

# (12) EUROPEAN PATENT APPLICATION

(21) Application number: 79301518.1

(22) Date of filing: 30.07.79

(51) Int. Cl.³: C 07 D 241/08, A 61 K 31/495 // A61K31/557

(30) Priority: 08.08.78 GB 3267878

(43) Date of publication of application: 20.02.80 Bulletin 80/4

(84) Designated Contracting States: AT BE CH DE FR GB IT LU NL SE

(71) Applicant: BEECHAM GROUP LIMITED, Beecham House Great West Road, Brentford, Middlesex (GB)

(72) Inventor: Wootton, Gordon, 51 Park Way, Sawbridgeworth Hertfordshire (GB)

(74) Representative: Dawson, Hugh Bainforde, Dr. et al, BEECHAM PHARMACEUTICALS Yew Tree Bottom Road, Epsom, Surrey KT18 5XQ. (GB)

## (54) Cyclic diamides, a process for their preparation and their pharmaceutical compositions.

(57) Compounds of the formula (I):

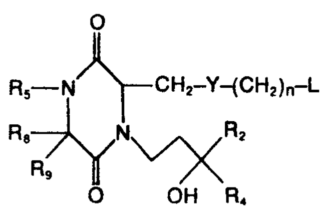

characterised in that

n is 1 to 5;

L is $CO_2R$ wherein R is hydrogen or $CO_2R$ is an ester group in which R contains from 1 to 12 carbon atoms, or $CH_2COR_1$ wherein $R_1$ is $C_{1-4}$ alkyl;

Y is $-CH_2CH_2-$, $-CH{=}CH-$ or $-C{\equiv}C-$;

$R_2$ is hydrogen or $C_{1-4}$ alkyl;

$R_4$ is hydrogen or $C_{1-9}$ alkyl, $C_{3-8}$ cycloalkyl, $C_{3-8}$ cycloalkyl-$C_{1-6}$ alkyl; or

$R_2$ and $R_4$ taken with the carbon atom to which they are joined represent a $C_{5-8}$ cycloalkyl group;

$R_5$ is $NR_6R_7$ wherein $R_6$ and $R_7$ independently are hydrogen, $C_{1-4}$ alkyl, phenyl $C_{1-4}$ alkyl or $NR_6R_7$ is a 3 to 7 membered heterocyclic group containing only one hetero atom; hydrogen, $C_{1-4}$ alkyl or $C_{1-4}$ alkyl substituted by an OH, $C_{1-4}$ alkoxy, CN, halogen or $NR_6R_7$ group as defined above, or by one or two $CO_2A$ groups in which A is hydrogen or a group containing from 1 to 12 carbon atoms; and $R_8$ and $R_9$ independently are hydrogen, $C_{1-4}$ alkyl or together with the carbon atom to which they are joined are a $C_{3-6}$ cycloalkyl group; and salts thereof, having activities similar to but more selective than those of natural prostaglandins, a process for their preparation and pharmaceutical compositions containing them.

EP 0 008 186 A1

# Cyclic Diamides

This invention relates to novel compounds having pharmacological activity, to a process for their preparation, to intermediates useful in that process and to pharmaceutical compositions containing them.

German Offenlegungsschrift No. 2552312 discloses that compounds of the formula (I)":

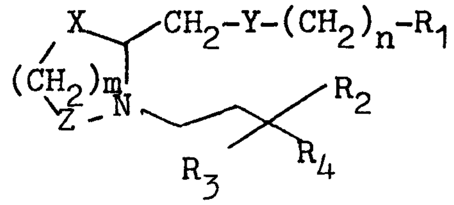

(I)"

wherein:

X is CO, protected CO, CROH in which R is hydrogen or $C_{1-4}$ alkyl and in which the OH moiety may be protected; Y is $CH_2CH_2$ or CH=CH; Z is CO or $CH_2$; n is 1 to 8; m is 1, 2 or 3; $R_1$ is hydrogen, $CH_2OH$, $CH_2OH$ in which the OH moiety is protected, $CO_2W$ wherein W is hydrogen or $CO_2W$ represents an ester group in which the ester moiety contains from 1 to 12 carbon atoms, or $CONH_2$; $R_2$ is hydrogen, $C_{1-4}$ alkyl, or taken together with $R_3$ and the carbon atom to which it is attached represents a carbonyl group; $R_3$ is hydrogen, hydroxy or protected hydroxy; $R_4$ is hydrogen or $C_{1-9}$ alkyl; and salts thereof; have useful pharmacological activity.

German Offenlegungsschrift No. 2755711 discloses that compounds of the formula (II)":

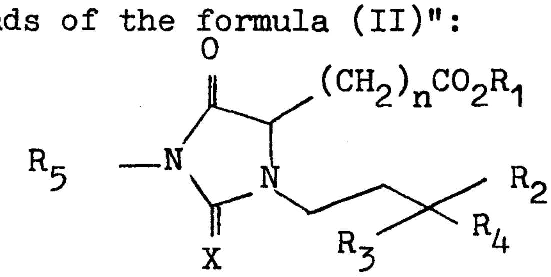

(II)"

wherein:

X is O or S;

n is 1 to 8;

$R_1$ is hydrogen, or $CO_2R_1$ represents an ester group in which the $R_1$ moiety contains from 1-12 carbon atoms;

$R_2$ is hydrogen, $C_{1-4}$ alkyl, or phenyl;

$R_3$ is hydroxy or protected hydroxy;

$R_4$ is hydrogen, $C_{1-9}$ alkyl, $C_{3-8}$ cycloalkyl, $C_{3-8}$ cycloalkyl-$C_{1-6}$ alkyl, phenyl, phenyl-$C_{1-6}$ alkyl, naphthyl,naphthyl-$C_{1-6}$-alkyl, any of which phenyl or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups; or

$R_2$ and $R_4$ taken with the carbon atom to which they are joined represent a $C_{5-8}$ cycloalkyl group;

$R_5$ is hydrogen, $C_{1-6}$ alkyl, $C_{1-6}$ alkyl substituted by a nitro, hydroxy, $C_{1-6}$ alkoxy, $CO_2A$, $(CO_2A)_2$, CN or halogen group, $C_{5-8}$ cycloalkyl, phenyl, phenyl-$C_{1-6}$ alkyl, phenyl-$C_{3-6}$ cycloalkyl, any of which phenyl moieties may be substituted by one or more halogen, trifluoromethyl, $C_{1-6}$ alkyl, $C_{1-6}$ alkoxy or nitro groups; or a group $CO_2A$; in $R_5$ when present A is hydrogen or $CO_2A$ represents an ester group in which the A moiety contains from 1 to 12 carbon atoms; and salts thereof; have similarly useful pharmacological activity.

A novel class of compounds also having useful pharmacological activity has now been discovered, which compounds are structurally distinct from the prior art referred to above.

Accordingly, the present invention provides a compound of the formula (I):

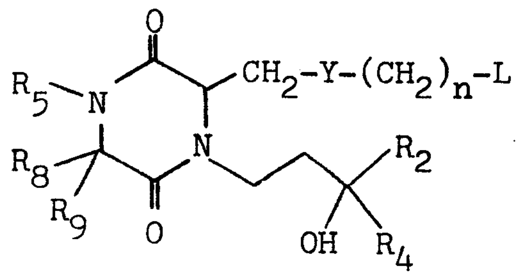

(I)

wherein:

n is 1 to 5;

L is $CO_2R$ wherein R is hydrogen or $CO_2R$ is an ester group in which R contains from 1 to 12 carbon atoms; or $CH_2COR_1$ wherein $R_1$ is $C_{1-4}$ alkyl;

Y is $-CH_2CH_2-$, $-CH=CH-$ or $-C{\equiv}C-$;

$R_2$ is hydrogen or $C_{1-4}$ alkyl;

$R_4$ is hydrogen or $C_{1-9}$ alkyl, $C_{3-8}$ cycloalkyl, $C_{3-8}$ cycloalkyl-$C_{1-6}$ alkyl; or

$R_2$ and $R_4$ taken with the carbon atom to which they are joined represent a $C_{5-8}$ cycloalkyl group;

$R_5$ is $NR_6R_7$ wherein $R_6$ and $R_7$ independently are hydrogen, $C_{1-4}$ alkyl, phenyl-$C_{1-4}$ alkyl or $NR_6R_7$ is a 3 to 7 membered heterocyclic group containing only one hetero atom; hydrogen, $C_{1-4}$ alkyl or $C_{1-4}$ alkyl substituted by an OH, $C_{1-4}$ alkoxy, CN, halogen or $NR_6R_7$ group as defined above, or by one or two $CO_2A$ groups in which A is hydrogen or a group containing from 1 to 12 carbon atoms; and

$R_8$ and $R_9$ independently are hydrogen, $C_{1-4}$ alkyl or together with the carbon atom to which they are joined are a $C_{3-6}$ cycloalkyl group;

and salts thereof.

n is preferably 3.

When L is $CO_2R$, R is hydrogen or $CO_2R$ is an ester

group in which R contains from 1 to 12 carbon atoms. Examples of R include hydrogen, methyl, ethyl, n- and iso-propyl, n-, sec- and tert-butyl, phenyl, benzyl, tolyl and the like, whilst normally hydrogen or $C_{1-6}$ alkyl groups are preferred.

When L is $CH_2COR_1$, $R_1$ is $C_{1-4}$ alkyl. Examples of $R_1$ include methyl, ethyl, n- and iso-propyl and n-, sec- and tert-butyl, preferably methyl and ethyl, in particular methyl.

Suitable examples of $R_2$ include hydrogen, methyl and ethyl, preferably methyl.

Suitable groups $R_4$ when $R_4$ is a $C_{1-9}$ alkyl group include $C_{4-9}$ alkyl groups. Such $C_{4-9}$ alkyl groups may be straight chain alkyl groups, such n-butyl, n-pentyl, n-hexyl and n-heptyl, or may be alkyl groups branched by one or two methyl groups (at the same or different carbon atoms). Thus for example, $R_4$ may be a group $CH_2$ M $CH(CH_3)M$ or $C(CH_3)_2$ M wherein M is a straight chain alkyl group such that the carbon content of the resultant group $R_4$ is 4 to 9.

In general preferred groups $R_4$ when $R_4$ is a $C_{4-9}$ alkyl group include straight chain pentyl, hexyl and heptyl groups. Of these, straight chain hexyl is often the most useful. Other preferred groups $R_4$ include groups $CH(CH_3)M$ and $C(CH_3)_2M$ wherein M is straight chain butyl, pentyl and hexyl.

Other suitable examples of $R_4$ when $R_4$ is an alkyl group include the lower alkyl groups, that is when $R_4$ is a $C_{1-4}$ alkyl group.

When $R_4$ is or contains a $C_{3-8}$cycloalkyl moiety, the moiety may be cyclopropyl. The moiety is preferably a $C_{5-8}$ cycloalkyl moiety such as a cyclohexyl moiety.

When $R_4$ is thus preferably $C_{5-8}$ cycloalkyl or $C_{5-8}$ cycloalkyl-$C_{1-6}$alkyl, it may be represented by a group of formula (II):

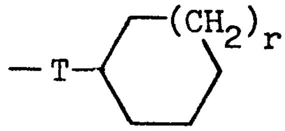

(II)

wherein r is 0 to 3; and

T is a bond or $C_{1-6}$ alkylene.

r is suitably 1.

When T is $C_{1-6}$ alkylene it may be straight-chain or branched. If branched it is preferably branched by one or two methyl groups at the same or different carbon atoms.

Examples of suitable T when $C_{1-6}$ alkylene are groups derived from substitution of the corresponding $C_{1-6}$ alkyl group and include those derived from methyl, ethyl, propyl, butyl and pentyl.

Often T is a group $-(CH_2)_q-$ wherein q is 0 to 4.

Also $R_2$ and $R_4$ taken with the carbon atom to which they are joined can represent a $C_{5-8}$ cycloalkyl group, such as a cyclohexyl group.

When $R_5$ is or contains an acyclic group $NR_6R_7$, $R_6$ and $R_7$ are suitably the same, and may advantageously be hydrogen. However, other suitable examples of such $NR_6R_7$ include those wherein one or both of $R_6$ and $R_7$ are methyl, ethyl and n- and iso-propyl and benzyl.

When $R_5$ is or contains a heterocyclic group $NR_6R_7$, suitable examples of such $NR_6R_7$ include the ring 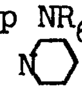.

When $NR_6R_7$ is a heterocyclic group, $R_5$ is more suitably $C_{1-4}$ alkyl substituted by $NR_6R_7$, preferably $CH_2NR_6R_7$.

$R_5$ may also be hydrogen or $C_{1-4}$ alkyl. When $R_5$ is $C_{1-4}$ alkyl suitable examples of $R_5$ include methyl, ethyl, n- and iso-propyl, n-, sec- and tert-butyl, more suitably methyl or ethyl, preferably methyl.

$R_5$ may also be a $C_{1-4}$ alkyl group substituted by an OH, $C_{1-4}$ alkoxy, CN, halogen or $NR_6R_7$ group as defined, or by one or two $CO_2A$ in which A is a group containing from 1 to 12 carbon atoms.

In such cases $R_5$ will often be a methyl group so substituted.

Suitable examples of $C_{1-4}$ alkoxy in $R_5$ include methoxy.

When $R_5$ contains a group $CO_2A$, suitable examples of A include hydrogen, methyl, ethyl, n- and iso-propyl, n-, sec- and tert-butyl, phenyl, benzyl, tolyl and the like, whilst normally hydrogen or $C_{1-6}$ alkyl are preferred.

When $R_8$ and $R_9$ are hydrogen or $C_{1-4}$ alkyl, they are suitably the same, and may advantageously be hydrogen.

When one or both $R_8$ and $R_9$ are $C_{1-4}$ alkyl, suitable examples are methyl, ethyl, n- and iso-propyl and n-butyl, more suitably methyl or ethyl.

$R_8$ and $R_9$ taken with the carbon atom to which they are joined can also represent a $C_{3-6}$ cycloalkyl group, such as a cyclopropyl group.

The compounds of the formula (I) may form conventional salts. Such salts include those with alkali and alkaline earth metals, suitably the alkali metals, more suitably sodium or potassium, and also include ammonium and substituted ammonium salts, and acid addition salts when $R_5$ is or contains $NR_6R_7$.

The group L when it is $CO_2H$ may be salified. Salts with the alkali metals may also be formed by the displacement of $R_5$ when hydrogen by an alkali metal atom.

From the aforesaid it will be seen that one particularly suitable group of compounds within formula (I) consists of those of formula (III):

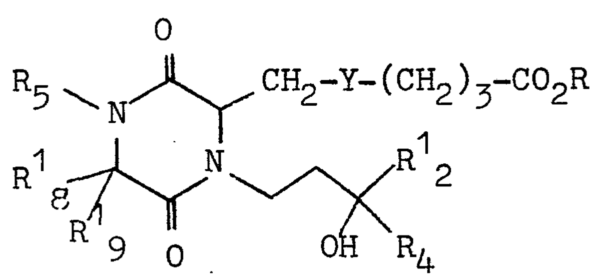

(III)

wherein:

Y, R, $R_4$ and $R_5$ are as defined in formula (I);

$R^1{}_2$ is hydrogen, methyl or ethyl;

$R^1{}_8$ and $R^1{}_9$ are the same and are hydrogen, $C_{1-4}$ alkyl or together with the carbon atom to which they are joined are a $C_{3-6}$ cycloalkyl group;

and salts thereof.

Examples of R and preferred R are as so described under formula (I).

$R^1{}_2$ is more suitably hydrogen or methyl, preferably methyl.

In formula (III) when $R_4$ is a $C_{1-9}$ alkyl group it is normally a $C_{4-9}$ alkyl group. Preferred groups $R_4$ when $R_4$ is a $C_{4-9}$ alkyl group are as so described under formula (I).

When $R_4$ is or contains a $C_{3-8}$ cycloalkyl moiety, the moiety is preferably $C_{5-8}$ cycloalkyl, so that preferred $R_4$ is of formula (II) in this case.

Suitable and preferred $R_4$ of formula (II) are as so described under formula (II).

Similarly, suitable and preferred $R_5$ are as so described under formula (I).

Suitable and preferred $R^1{}_8$, $R^1{}_9$ are as so described for $R_8$, $R_9$ under formula (I).

Another particularly suitable group of compounds within formula (I) consists of those of formula (IV):

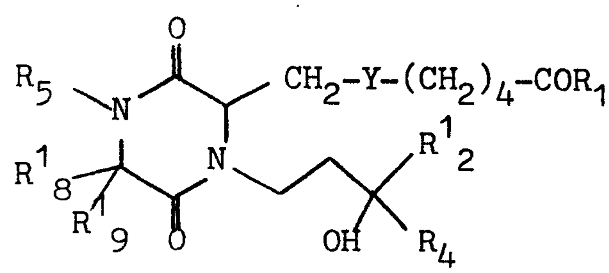

(IV)

wherein:

$R_1$ is $C_{1-4}$ alkyl and the remaining variables are as defined in formula (III), and salts thereof.

In formula (IV) examples of $R_1$ and preferred $R_1$ are as so described under formula (I).

Examples of the remaining variable groups in formula (IV) and suitable and preferred of these variable groups are as so described under formula (III).

An interesting group of compounds within formula (I) consists of those of formula (V) and salts thereof:

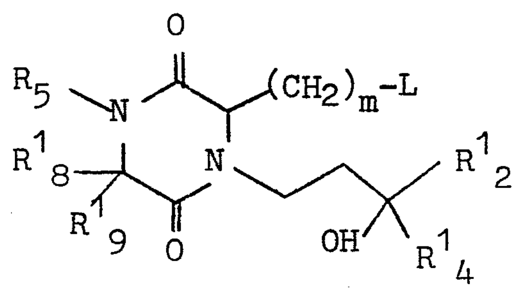

(V)

wherein:

m is 5, 6 , 7 or 8;

$R_4^1$ is hydrogen or $C_{1-9}$ alkyl;

L and $R_5$ are as defined in formula (I); and the remaining variables are as defined in formula (III).

In formula (V) m is preferably 6.

Whilst $R^1_4$ may be hydrogen or a $C_{1-9}$ alkyl, it is normally a $C_{4-9}$ alkyl group. In such cases, suitable and preferred groups $R^1_4$ include those previously so described for the group $R_4$ when $R_4$ is a $C_{4-9}$ alkyl group. Of these, straight chain hexyl is often the most useful. Other preferred groups $R^1_4$ include $CH(CH_3)$ M and $C(CH_3)_2$ M wherein M is straight-chain butyl, pentyl or hexyl.

Examples of the remaining variable groups in formula (V) and suitable and preferred of these variable groups are as so described under formula (III).

Another interesting group of compounds within formula (I) consists of those of formula (VI):

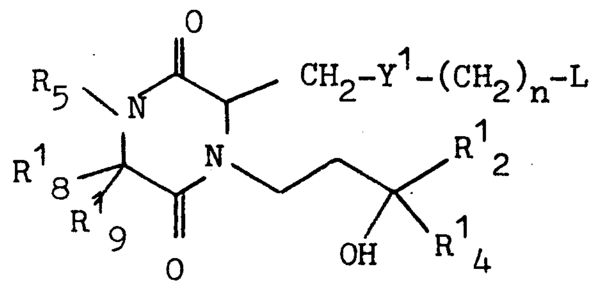

(VI)

wherein:

$Y^1$ is -CH=CH- or -C≡C-;

n, L and $R_5$ are as defined in formula (I);

$R^1{}_4$ is as defined in formula (V), and the remaining variables are as defined in formula (III); and salts thereof.

In formula (VI) n is preferably 3.

$R^1{}_4$ is normally a $C_{4-9}$ alkyl group. Suitable and preferred $C_{4-9}$ alkyl groups $R^1{}_4$ are as so described under formula (V). In formula (VI) straight-chain hexyl is again often the most useful.

Examples of the remaining variable groups in formula (VI), and suitable and preferred of these variable groups, are as so described under formula (III).

A further interesting group of compounds within formula (I) consists of those of formula (VII):

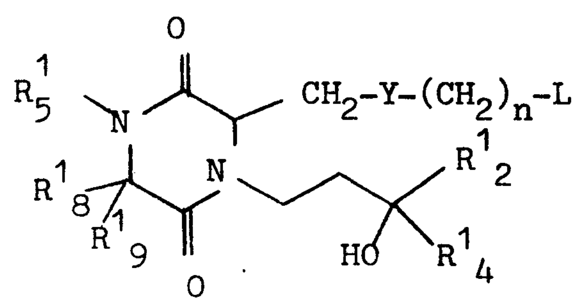

(VII)

wherein $R_5{}^1$ is hydrogen or $C_{1-4}$ alkyl, and the remaining variables are as defined in formula (III); and salts thereof.

$R^1_5$ is suitably hydrogen, methyl or ethyl, preferably hydrogen or methyl.

In formula (VII) n is preferably 3.

$R^1_4$ is normally a $C_{4-9}$ alkyl group. Suitable and preferred $C_{4-9}$ alkyl groups $R^1_4$ are as so described under formula (V). In formula (VII) straight chain hexyl is again often the most useful.

Examples of the remaining variable groups in formula (VII) and suitable and preferred of these variable groups are as so described under formula (III).

A preferred group of compounds within formula (I) is those of formula (XI):

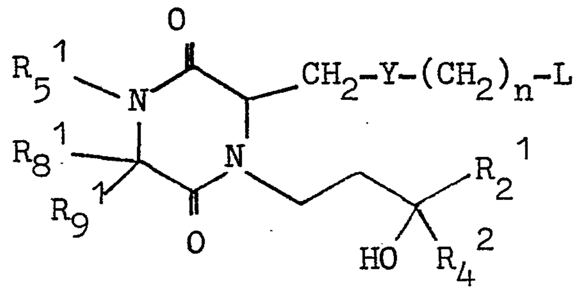

(XI)

wherein $R^2_4$ is $C_{3-8}$ cycloalkyl, or $C_{3-8}$ cycloalkyl $C_{1-6}$ alkyl and the remaining variables are as defined in formula (VII), and salts thereof.

$R_4^2$ is preferably $C_{5-8}$ cycloalkyl, in particular cyclohexyl.

n is preferably 3.

L is preferably a group $CO_2R$ as defined in formula (I).

Y is preferably $-CH_2CH_2-$.

Suitable and preferred remaining variable groups are as so described under formula (III).

A preferred group of compounds within those of formula (XI) are of formula (XII):

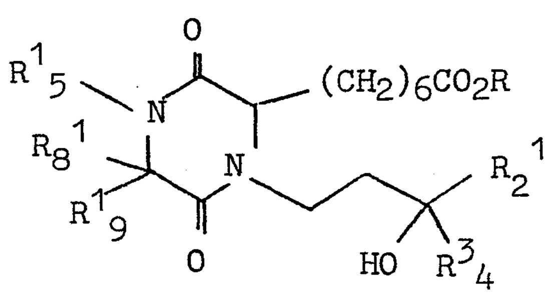

(XII)

wherein $R_4^3$ is a group of formula (II):

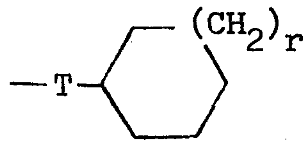

wherein r is 0 to 3; and T is a bond or $C_{1-6}$ alkylene; and the remaining variables are as defined in formula (XI), and salts thereof.

$R^2_4$ is preferably cyclohexyl.

$R^1_5$ is preferably methyl.

Other suitable and preferred variable groups are as so described under formula (III).

This invention also provides a process for the preparation of compounds of the formula (I), which process comprises cyclising a compound of formula (VIII):

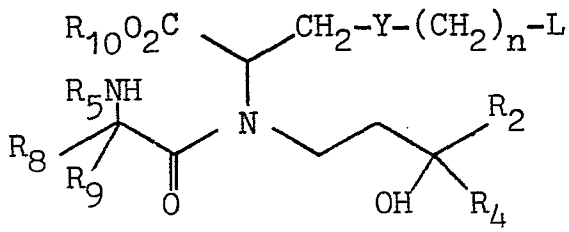

(VIII)

wherein:

$R_{10}$ is hydrogen or $CO_2R_{10}$ is an ester group in which $R_{10}$ contains from 1 to 12 carbon atoms; and the remaining variables are as previously defined; with elimination of $R_{10}OH$; and thereafter as desired converting R, $R_5$, $R_6$, $R_7$ and/or Y in the compound of formula (I) thus formed into other R, $R_5$, $R_6$, $R_7$ or Y.

The cyclisation is generally effected by warming, for instance . in an inert solvent such as benzene. It should be noted that, when $R_5$ is a sterically hindering group, the reaction may require effecting with a strong base in a dry organic solvent, such as sodium hydride or sodium ethoxide in benzene or potassium t-butoxide in toluene, benzene or hexamethylphosphoramide.

In general it is convenient in this reaction that when L is $CO_2R$ as defined above, this group is an ester group as defined.

Often it is convenient if R and $R_{10}$ are the same.

The conversion of a compound of the formula (I) wherein $CO_2R$ is an ester group to one wherein R is hydrogen may be achieved by conventional de-esterification. Similarly, compounds wherein R is hydrogen may be esterified conventionally.

When L is a group $CO_2H$ in a compound of the formula (I), salts of the compound at that carboxyl group may be formed conventionally, for example by reacting the compound

with a relevant base.

In general it is convenient that $R_5$ is hydrogen in the compounds of formula (VIII) and $R_5$ in corresponding compounds of the formula (I) is converted to different $R_5$ by conventional substitution.

However, in the compounds of formula (VIII) $R_5$ may also conveniently be $C_{1-4}$ alkyl.

When $R_5$ = hydrogen in compounds of formula (VIII) is converted to other $R_5$ by conventional substitution, this may be carried out by reaction with $R_5Q$ wherein Q is a readily displaceable good leaving group. In such cases, it may often be necessary to convert the compound of the formula (I) wherein $R_5$ is hydrogen to an alkali metal salt at the lactam group bearing $R_5$, for example by reacting the compound with a relevant base in a suitable solvent. The base should be a strong base such as sodium in an alcohol such as ethanol, sodium hydride in dimethylformamide or lithium di-isopropylamide in hexamethylphosphoramide.

Suitable examples of Q will be well-known to the skilled man and include for example halides, such as bromide, when $R_5$ is $C_{1-4}$ alkyl or substituted $C_{1-4}$ alkyl as defined; or substituted aryloxy, such as 2,4-dinitrophenoxy, when $R_5$ is $NR_6R_7$ as defined.

The skilled man will realise that substituting an $R_5$ hydrogen sometimes also substitutes an R hydrogen.

Thus, if it is desired to form a compound of the formula (I) wherein R is hydrogen and $R_5$ is not by substitution of a compound of the formula (I) wherein $R_5$ is hydrogen, then it is preferable that $CO_2R$ is initially an ester group which is de-esterified conventionally after the substitution of $R_5$ hydrogen.

When $R_5$ is $NR_6R_7$ or $C_{1-4}$ alkyl substituted by $NR_6R_7$, the skilled man will realise that, if a compound of the formula (I) is desired wherein $R_6$ and $R_7$ are both hydrogen,

then it is frequently desirable to protect the amino group so defined during reaction by means of conventional amine protecting groups $R_6$ and $R_7$, such as benzyl groups. Such groups are readily removable by conventional methods such as hydrogenolysis.

Similarly, groups $NR_6R_7$ wherein $R_6$ and $R_7$ are hydrogen may be alkylated or aralkylated conventionally.

When $R_5$ is or contains a group $NR_6R_7$ in a compound of the formula (I), acid addition salts of the compound at that group may be formed conventionally, for example by reacting the compound with a relevant acid.

Compounds wherein Y is -C≡C- may be reduced to compounds wherein Y is -CH=CH- in known manner, suitably by catalytic hydrogenation, for example using Lindlar catalysis.

Similarly when Y is -CH=CH-, it may be reduced to $-CH_2CH_2-$ in known manner, suitably by catalytic hydrogenation, for example, using transition metals.

The compounds of formula (VIII) may be prepared by reacting a compound of formula (IX):

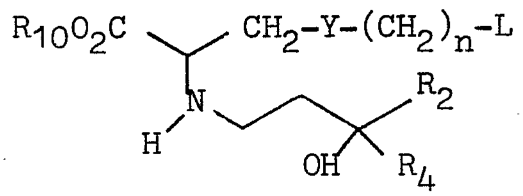

(IX)

wherein the variables are as previously defined, with a compound of formula (X):

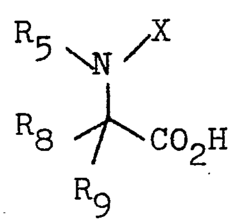

(X)

wherein:

X is a conventional amine protecting group; and the remaining variables are as previously defined; or with

such a compound containing a reactive derivative of the $CO_2H$ group thereof; and thereafter replacing X conventionally by a hydrogen atom.

Conventional amine protecting groups are those which are readily removeable by conventional mild methods such as mild hydrolysis or hydrogenolysis. Examples of such groups include benzyloxycarbonyl.

Suitable reactive derivatives of the $CO_2H$ group include the corresponding halides or anhydrides.

Where the free acid (X) is used the reaction is preferably carried out in the presence of a condensation promoting dehydrating agent such as dicyclohexylcarbodiimide.

X is suitably removed from the compound so formed by mild hydrogenolysis, for example using hydrogen in the presence of a transition metal catalyst such as palladium on charcoal.

The compound of the formula (VIII) is often conveniently formed *in situ* by the above process, and cyclised directly to the corresponding compound of the formula (I) without isolation.

It is believed that the compounds of formula (VIII) are novel, and thus form an important aspect of this invention as intermediates.

The compounds of formula (IX) may be prepared by the method disclosed in German Offenlegungsschrift Nos: 2552312, 2647969 and 2724948 and our co-pending European Application No. 79301145.3.

The compounds of the formula (I) have asymmetric centres, and can thus exist in several sterisomeric forms. The invention extends to each of these forms, and to mixtures thereof (including racemates). The different stereoisomeric forms may be separated mutually by usual methods.

Isomers having a common stereospecific feature may be obtained by stereospecific synthesis. Isomers having a common chiral centre may be obtained by asymmetric synthesis, e.g. from chiral intermediates.

Compounds of the formula (I) have useful pharmacological activity. For example compounds of the formula (I) have anti-gastric secretion activity e.g. anti-ulcer activity, cardiovascular activity e.g. antihypertensive activity, platelet aggregation inhibition activity, affect the respiratory tract e.g. bronchodilator activity, anti-fertility activity, smooth muscle activity and/or anti-arrhythmic activity.

In general it may be said that compound within the formula (I) have a range or pharmacological activities similar to those shown by the natural prostaglandins, but that their activity profiles tend to be rather more selective so that each compound tends to have a major activity readily ascertained by routine pharmacological tests. By way of example, it has been found that many of the compounds fo the formula (I) are especially useful as bronchodilator agents, such as in particular compounds of the fomulae (V) and (XI).

The invention therefore also provides a pharmaceutical composition comprising a compound of the formula (I) and a pharmaceutically acceptable carrier.

In order to utilise the selectivity of activity found with compounds of the formula (I), normally a given compound will be used in the treatment of the disorder corresponding to the compound's major activity (that is, the disorder for which the compound has the lowest active dose) and will accordingly be formulated into the corresponding pharmaceutical composition, and administered in a manner conventional for treatment of that disorder. It may also of course be possible with compounds having one or more further pronounced activities to formulate and use the compound for those further activities as well as for the major activity, provided that there is no undesirable pharmacological interaction between the different activities, or that separation of the different activities can be obtained by a difference in the formulation or in the mode of administration.

The compositions may be in the form of tablets, capsules, powders, granules, lozenges or liquid preparations, such as oral or sterile parenteral solutions or suspensions.

Tablets and capsules for oral administration may be in unit dose presentation form, and may contain conventional excipients such as binding agents, fillers, tabletting lubricants, disintergrants, and acceptable wetting agents and the like. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, emulsifying agents, non-aqueous vehicles (which may include edible oils), preservatives, and if desired conventional flavouring or colouring agents, and the like.

For parenteral administration, fluid unit dosage forms are prepared utilizing the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilization cannot be accomplished by

filtration. The compound can be sterilized by exposure to ethylene oxide before suspensing in the sterile vehicle. Advantageously, a sufactant or wetting agent is included in the composition to facilitate uniform distribution of the compound.

When appropriate, the compositions of this invention may be presented as an aerosol for oral administration, or as a microfine powder for insufflation.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The invention also provides a method of treatment and/or prophylaxis of disorders in human beings or domestic animals which comprises the administration to the sufferer of an effective amount of a compound of the formula (I).

The following Example illustrates the preparation of compounds of the formula (I).

The following Description illustrates the preparation of intermediates for the compounds of the formula (I).

DESCRIPTION

Dimethyl 2-[N-(3'-hydroxy-3'-methylnonyl)-N-{2"-(N'-methyl-N'-benzyloxycarbonylamino)acetyl}amino]azelate.

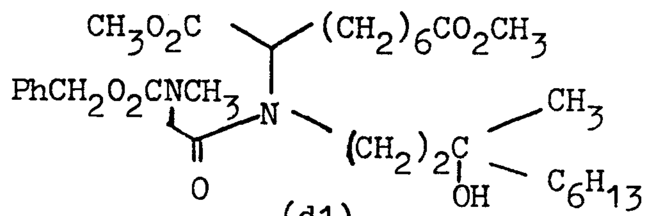

(d1)

Dicyclohexyl carbodiimide (10.65g) in dichloromethane (50ml) was added dropwise, under nitrogen, at room temperature, to a stirred mixture of N-benzyloxycarbonyl sarcosine (11.53g) and dimethyl 2-[N-(3'-hydroxy-3'-methyl)nonyl]aminoazelate (20g) in dichloromethane (200ml). The mixture was stirred at room temperature overnight then was filtered through kieselguhr. The filtrate was chromatographed on silica gel (600g) using chloroform as eluent. Dimethyl 2-[N-(3'-hydroxy-3'-methylnonyl)-N-{2"-(N'-methyl-N'-benzyloxycarbonylamino) acetyl}amino] azelate (19.5g) was obtained as a colourless gum

I.r: $\nu_{max}$ (film) $cm^{-1}$ 3450, 1740, 1710, 1660.

The compounds shown in Table 1 were produced in a similar way, using the appropriate intermediates (A and B).

Table 1

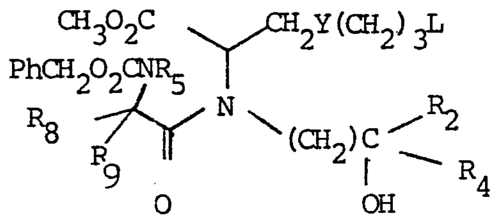

| COMPOUND | Intermediate A | | | Intermediate B | | | |
|---|---|---|---|---|---|---|---|
| | 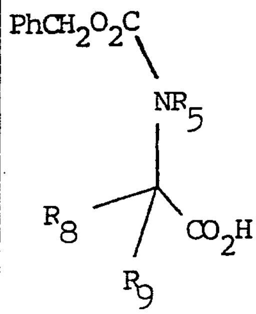 | | | 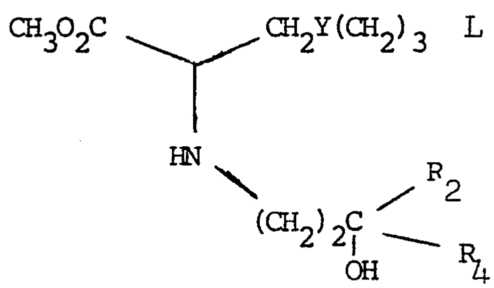 | | | |
| | $R_5$ | $R_8$ | $R_9$ | $R_2$ | $R_4$ | Y | L |
| d2 | H | H | H | $CH_3$ | $C_6H_{13}$ | $CH_2CH_2$ | $CO_2CH_3$ |
| d3 | $CH_3$ | H | H | $CH_3$ | $CH(CH_3)C_4H_9$ | $CH_2CH_2$ | $CO_2CH_3$ |
| d4 | $CH_3$ | H | H | $CH_3$ | $C_2H_5$ | $CH_2CH_2$ | $CO_2CH_3$ |
| d5 | $CH_3$ | H | H | $CH_3$ | 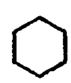 | $CH_2CH_2$ | $CO_2CH_3$ |
| d6 | H | H | H | $CH_3$ | 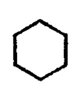 | $CH_2CH_2$ | $CO_2CH_3$ |

| | $R_5$ | $R_8$ | $R_9$ | $R_2$ | $R_4$ | Y | L |
|---|---|---|---|---|---|---|---|
| d 7 | H | H | H | H | $C_2H_5$ | $CH_2CH_2$ | $CO_2CH_3$ |
| d 8 | H | H | H | 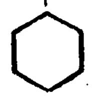 | | $CH_2CH_2$ | $CO_2CH_3$ |
| d 9 | $CH_3$ | H | H | $CH_3$ | $(CH_2)_2$ 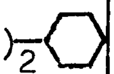 | $CH_2CH_2$ | $CO_2CH_3$ |
| d 10 | H | $CH_3$ | H (L-isomer) | $CH_3$ | 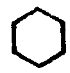 | $CH_2CH_2$ | $CO_2CH_3$ |
| d 11 | $CH_3$ | H | H | $CH_3$ | $C_6H_{13}$ | $CH_2CH_2$ | $CH_2COCH_3$ |

EXAMPLE 1

1-(3'-hydroxy-3'-methylnonyl)-4-methyl-6-(6"-methoxycarbonylhexyl) piperazine-2,5-dione.

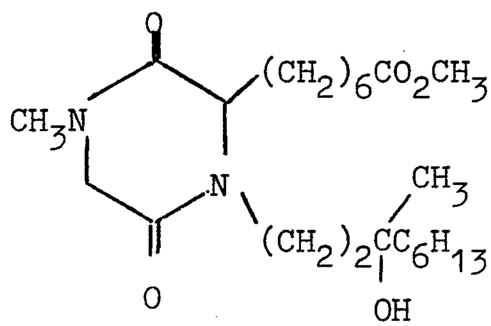

(I)

Dimethyl 2-[N-(3'-hydroxy-3'-methylnonyl)-N-{2"-(N-methyl-N'-benzyloxycarbonylamino)acetyl}amino]azelate (15g) was hydrogenolysed, at atmospheric pressure and room temperature, over 10% palladium on charcoal (3g) in dimethoxyethane (300ml). When hydrogen uptake was completed, the mixture was filtered through kieselguhr and the filtrate was evaporated *in vacuo* to give a clear gum (9.5g). This was purified via column chromatography on silica gel (100g) using chloroform as eluant to give 1-(3'-hydroxy-3'-methylnonyl)-4-methyl-6-(6"-methoxycarbonylhexyl)-piperazine-2,5-dione (7.6g) as a colourless gum.

I.r. $\nu_{max}$ (film) $cm^{-1}$ 3450, 1740, 1660 (broad)

The compounds shown in Table 2 were prepared in a similar manner.

Table 2

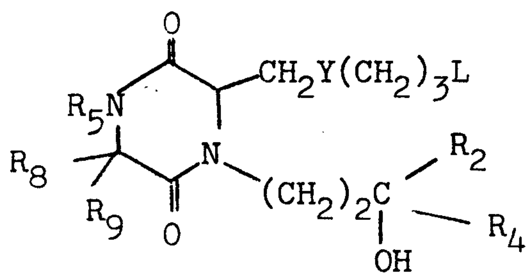

| Compound | $R_2$ | $R_4$ | $R_5$ | $R_8$ | $R_9$ | Y | L |
|---|---|---|---|---|---|---|---|
| 2 | $CH_3$ | $C_6H_{13}$ | H | H | H | $CH_2CH_2$ | $CO_2CH_3$ |
| 3 | $CH_3$ | $CH(CH_3)C_4H_9$ | $CH_3$ | H | H | $CH_2CH_2$ | $CO_2CH_3$ |
| 4 | $CH_3$ | 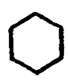 | $CH_3$ | H | H | $CH_2CH_2$ | $CO_2CH_3$ |
| 5 | $CH_3$ | 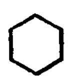 | H | H | H | $CH_2CH_2$ | $CO_2CH_3$ |

Table 2 (continued)

| Compound | $R_2$ | $R_4$ | $R_5$ | $R_8$ | $R_9$ | Y | L |
|---|---|---|---|---|---|---|---|
| 6 | H | $C_2H_5$ | H | H | H | $CH_2CH_2$ | $CO_2CH_3$ |
| 7 | 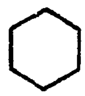 | | H | H | H | $CH_2CH_2$ | $CO_2CH_3$ |
| 8 | $CH_3$ | $(CH_2)_2$- 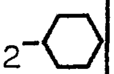 | $CH_3$ | H | H | $CH_2CH_2$ | $CO_2CH_3$ |
| 9 | $CH_3$ | 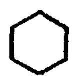 | H | $CH_3$ | H | $CH_2CH_2$ | $CO_2CH_3$ |
| 14 | $CH_3$ | $C_6H_{13}$ | $CH_3$ | H | H | $CH_2CH_2$ | $CH_2COCH_3$ |

Characterising Data

Compound 4

Analysis: $C_{23}H_{40}N_2O_5$ requires: C, 65.06; H, 9.50; N, 6.60%
found: C, 65.11; H, 9.18; N, 6.44%

I.r. $(CHCl_3)cm^{-1}$: 3450, (OH); 1735, $(CO_2CH_3)$; 1660 (CON<).

N.m.r.: $(CDCl_3)\tau$: 7.8 (bs, 1H, OH);
7.7 (m, 2H, $C\underline{H}_2CO_2CH_3$);
7.05 (s, 3H, $NCH_3$);
6.35 (s, 3H, $CO_2CH_3$);
6.2 to 5.8 (m, 3H, $COCH_2N$; NCH).

Mass spectrum: $C_{23}H_{40}N_2O_5$ (m*) requires: 424.2935
found: 424.2956

Compound 5

Analysis: $C_{22}H_{38}N_2O_5$ requires C, 64.36; H, 9.33; N, 6.82%
found: C, 64.57; H, 9.56; N, 6.45%

I.r. (ν max) ($CHCl_3$) $cm^{-1}$: 3450 to 3200, (OH; NH); 1735, ($CO_2CH_3$); 1680 and 1645 (CONH and CON<).

Mass spectrum: $C_{22}H_{38}N_2O_5$ (m*) requires: 410.2778
found: 410.2787

Compound 6

Analysis: $C_{17}H_{30}N_2O_5$ requires: C, 59.63; H, 8.83; N, 8.18%
found: C, 59.13; H, 8.70; N, 7.95%

N.m.r ($CDCl_3$)τ: 7.65 (t, 2H, $C\underline{H}_2CO_2CH_3$);
6.4 (centre of brm, 5H, CHOH, $NCH_2$, NCH);
6.3 (s, 3H, $CO_2CH_3$);
5.95 (bs, 2H, $NCH_2CON$);
2.65 (bs, 1H, CONH).

Mass spectrum $C_{17}H_{30}N_2O_5$ (m*) requires: 342.2155
found: 342.2157

Compound 7

Analysis: $C_{20}H_{34}N_2O_5$ requires: C, 62.80; H, 8.96; N, 7.32%
found: C, 62.69; H, 9.09; N, 7.29%

N.m.r. ($CDCl_3$)τ: 7.7 (t, 2H, $C\underline{H}_2CO_2CH_3$);
7.45 (s, 1H, OH);
6.85 (bm, 2H, $NCH_2$);
6.35 (s, 3H, $CO_2CH_3$);
6.05 (b, 3H, $NCH_2CON$; NCH);
2.6 (bs, 1H, CONH).

Mass spectrum: $C_{20}H_{34}N_2O_5$ (m*) requires: 382.2468
found: 382.2468

Compound 8

N.m.r. ($CDCl_3$) τ: 7.75 (t, 2H, C<u>H</u>$_2$CO$_2$CH$_3$);
7.72 (s, 1H, OH);
6.9 (centre of br m, 2H, NCH$_2$);
7.05 (s, 3H, NCH$_3$);
6.35 (s, 3H, CO$_2$CH$_3$);
6.1 (d, 2H, NCH$_2$CON);
5.95 (m, 1H, NCH).

Compound 9

I.r. (νmax) ($CHCl_3$) $cm^{-1}$ 3500 - 3200 (OH,NH);
1740, (CO$_2$CH$_3$);
1680 (CONH);
1640, (CON=).

N.m.r. ($CDCl_3$) τ: 7.7 (t, 2H, C<u>H</u>$_2$CO$_2$CH$_3$);
7.4 (bs, 1H, OH);
6.8 (centre of brm, 2H, NCH$_2$);
6.35 (s, 3H, CO$_2$CH$_3$);
6.0 (m, 2H, 2 x COCHN);
2.35 (b, 1H, CONH).

Compound 14

Analysis: $C_{24}H_{44}N_2O_4$ requires: C, 67.89; H, 10.44; N, 6.60%
found C, 67.97; H, 10.77; N, 6.51

I.r. ( max) ($CHCl_3$) $cm^{-1}$: 3440, (OH)
1720, (COCH$_3$)
1660, br, (CON)

N.m.r. ($CDCl_3$)τ: 7.9 (s, 3H, COCH$_3$);
7.6 (t, 2H, C<u>H</u>$_2$COCH$_3$);
7.5 (s, 1H, OH);
7.0 (s, 3H, NCH$_3$);

6.6 (centre bm, 2H, $CONCH_2$);
6.05 (m, 3H, ring $-CH_2-$, ring= CH)

Mass spectrum: $C_{24}H_{44}N_2O_4$ (m*)
requires: 424.3301
found: 424.3317

Example 2

1-(3'-hydroxy-3'-cyclohexylbutyl)-4-methyl-6-(6"-carboxy-hexyl)-piperazine-2,5-dione

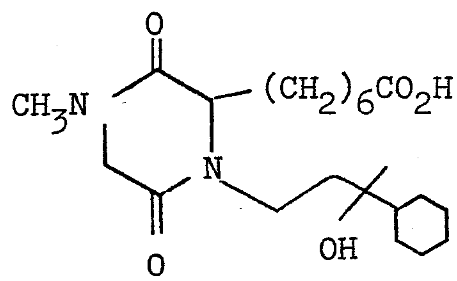

(10)

1-(3'-hydroxy-3'-cyclohexylbutyl)-4-methyl-6-(6"-methoxycarbonylhexyl)-piperazine-2,5-dione (4) (1.05g, 2.5 mmol) was dissolved in methanol (20ml) and 10% aqueous potassium carbonate (15 ml) was added. The mixture was stirred at room temperature for eighteen hours and partitioned between diethyl ether (3 x 50ml) and water (150 ml). The aqueous phase was acidified with 5M aqueous hydrochloric acid and extracted with diethyl ether (3 x 50 ml). The combined diethyl ether layers were washed with brine until neutral and dried over sodium sulphate. The solvent was removed under reduced pressure, and the yellow gum so obtained was recrystallised using ethyl acetate/hexane as solvent to give 1-(3'-hydroxy-3'-cyclohexylbutyl)-4-methyl-6-(6"-carboxyhexyl)-piperazine-2,5-dione (0.65g, 64%) as a white powder.

I.r. ($\nu$ max) $cm^{-1}$: 3425 (O-H), 3.50-2500 ($CO_2H$), 1720 ($CO_2HO$), 1660 (amide)

N.m.r. ($CDCl_3$)$\tau$: 3.89 (bs, 2H, O-H, $CO_2$-H exch $D_2O$); 7.06 (s, 3H, N-CH); 7.70 (2H, t, $C\underline{H}_2-CO_2H$)

Analysis: $C_{22}H_{38}N_2O_5$ requires : N, 6.82; C, 64.36; H, 9.33%
found: N, 6.68; C, 64.07; H, 9.47%

Example 3

1-(3'-hydroxy-3'-methylnonyl)-4-methoxycarbonylmethyl-6-(6"-methyoxycarbonylhexyl)-piperazine-2,5-dione

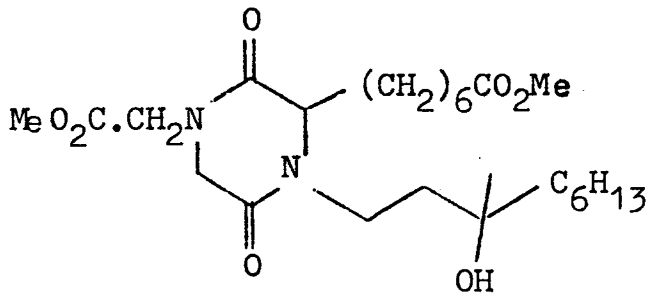

(11)

To a stirred slurry of sodium hydride (0.146g, 4.9 mmole) (80% dispersion in oil) in dry benzene (10ml) under a nitrogen atmosphere was added 1-(3'-hydroxy-3'-methylnonyl)-6-(6"-methoxycarbonylhexyl)-piperazine-2,5-dione (2.0g, 4.9 mmol) in dry dimethylformamide (10ml). After stirring for ten minutes, a clear colourless solution was obtained. Methyl bromoacetate (0.75g, 4.9 mmol) was added dropwise over half a minute. The solution was stirred at room temperature and then heated to reflux. A precipitate soon appeared. After three hours at reflux, the mixture was cooled, diluted with diethyl ether (200 ml) and washed with brine (3 x 40 ml). The solution was dried over sodium sulphate and the solvent was removed under reduced pressure. The oil so obtained was chromatographed on silica gel (30g) using ethyl acetate/hexane (1:1) and then ethyl acetate as eluant to give 1-(3'-hydroxy-3'-methylnonyl)-4-methoxy-carbonyl methyl---6-(6"-methoxycarbonylhexyl-piperazine-2,5-dione (1.25 g, 53%) as a low melting white solid.

I.r. ($cm^{-1}$) ($\nu$ max): 3425 (O-H), 1740 (ester), 1665 (amide).

N.m.r. ($CDCl_3$)$\tau$: 5.2-6.8 (m, 13H, $MeO_2$ C.$\underline{CH_2}$N-, $-\overset{O}{\overset{\|}{C}}-\underline{CH_2}-\overset{|}{N}-\overset{O}{\overset{\|}{C}}, \overset{O}{\overset{\|}{C}}-\overset{|}{C}H-N$, $-\overset{O}{\overset{\|}{C}}-\overset{|}{N}-CH_2$, 2 x $CO_2CH_3$); 7.15 (s, 1H, O-H exchange $D_2O$), 7.70(t, 2H, $CH_2\underline{CH_2}CO_2Me$)

H.r.m.s.: Found 484.3168, $C_{25}H_{44}N_2O_7$requires 484.3147

<u>1-(3'-hydroxy-3'-cyclohexylbutyl)-4-cyanomethyl-6-(6"-methoxycarbonylhexyl)-piperazine-2,5-dione</u>(12) was similarly prepared from 1-(3'-hydroxy-3'-cyclohexylbutyl)-6-(6"-methoxycarbonylhexyl)-piperazine-2,5-dione and chloroacetonitrile in 45% yield.

I.r. ($\nu$ max) $cm^{-1}$ 3450 (O-H), 1735 (ester), 1665 (amide).
N.m.r. ($CDCl_3$)$\tau$: 5.3-6.1 (bm, 5H, $NC-CH_2-N=$
$-\overset{\overset{O}{\|}}{C}-CH_2-\overset{|}{N}-\overset{\overset{O}{\|}}{C}-$ $\underset{\underset{O}{\|}}{C}-\underset{|}{C}H-N$); 6.36, (s, 3H, $CO_2CH_3$).

H.r.m.s.: Found 431.2784 ($M^+ - H_2O$); $C_{24}H_{37}N_3O_4$ requires 431.2782.

Example 4

1-(3'-hydroxy-3'-methylnonyl)-4-pyrrolidinomethyl-6-(6"-methoxycarbonylhexyl)-piperazine-2,5-dione

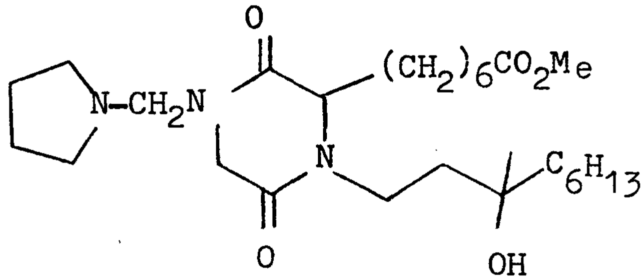

(13)

A mixture of 1-(3'-hydroxy-3'-methylnonyl)-6-(6"-methoxycarbonylhexyl)-piperazine-2,5-dione(1.4g, 3.4 mmol), 40% aqueous formaldehyde (0.29 ml 3.9 mmol) and pyrrolidine (0.27g, 3.8 mmol) in ethanol (10ml) was heated to reflux for eighteen hours. The resulting yellow solution was cooled, diluted with diethyl ether (200 ml) and washed with brine (3 x 40 ml). After drying over sodium sulphate, the solvent was removed under reduced pressure, and the dark yellow oil so obtained was chromatographed on neutral alumina (25g) using chloroform as eluent. This gave 1-(3'-hydroxy-3'-methylnonyl)-4-pyrrolidinomethyl-6-(6"-methoxycarbonylhexyl)-piperazine-2,5-dione (1.1g 65%).

I.r. ($\nu$ max) $cm^{-1}$: 3430 (O-H), 1740 (ester), 1660 (amide).

N.m.r. ($CDCl_3$)$\tau$: 5.5 - 6.1 (5H, N-$\underline{CH_2}$-N; $-\overset{O}{\overset{\|}{C}}-CH_2-\overset{|}{N}-\underset{\underset{O}{\|}}{C}-$ ; $-\overset{O}{\overset{\|}{C}}-\overset{|}{C}H-N$), 6.36 (s, 3H, $CO_2CH_3$); 7.40 (bm, 4H, 2 x $CH_2N$).

H.r.m.s.: Found 495.3680, $C_{27}H_{49}N_3O_5$ requires 495.3670

PHARMACOLOGICAL DATA

Bronchodilation Activity

1. The compounds were examined for their ability to inhibit 5-hydroxytryptamine or histamine induced bronchoconstriction in the anaesthetised, artifically respired guinea pig (Konzett-Rossler preparation). The compounds were administered intravenously. The results are shown in Table A.

Table A

| Compound number | $ED_{50}$ as defined above ($\mu g$/kg i.v.) |
|---|---|
| 1 | 4.4 |
| 3 | 4.3 |
| 4 | 0.59 |

2. Compound 3 was also examined for its ability to protect conscious guinea pigs against bronchoconstriction induced by an histamine aerosol (Herxheimer test). In these experiments the compound was administered by aerosol or by oral administration. The results are the mean of several experiments. Compound 3 was active at 10 mg/kg.

Anti-gastric sectory activity

The compounds were examined for their ability to inhibit pentagastrin-stimulated gastric acid secretion in the anaesthetised, perfused rat stomach preparation (Ghosh and Schild preparation). The compounds were administered intravenously.

Compounds 3 and 9 inhibited gastric acid secretion at 0.2 and 0.25 mg/kg, i.v., respectively.

Anti-ulcer activity

Anti-ulcer activity was assessed by the inhibition of indomethacin induced gastric damage in the rat according to the method of Eleghe (1974) Israeli J. Med. Sci. 10 1451. Rats were starved overnight given 15mg/kg indomethacin subcutaneously and sacrificed 4 hours later. Stomachs were reflated with n. saline, cut along the greater curvature pinned out and scored for gastric damage by the following system:

Score 1 - 3 - according to degree of erythema and slight haemorrhage.

Score 4 - 6 - according to degree of mucosal erosion.

Score 7 - 9 - according to depth of gastric damage.

Groups of 7 rats were used for each treatment and the test compound or vehicle were administered 30 minutes prior to giving the indomethacin. Dose of test compound was 50mg/kg orally and control groups receiving vehicle only were set up simultaneously. Mean values for each treatment were obtained using the above scoring system and the Mann Witney test applied for significance of difference between the values obtained with the treatments.

The results are shown in Table B

Table B

| Compound number | Vehicle Mean Score ± S.E. of Mean | Test Mean Score ± S.E. of Mean |
|---|---|---|
| 3 | 4.57 ± 0.69 | 0.71 ± 0.42 ($p < 0.01$) |

Toxicity

No toxic effects were observed in any of the above tests.

CLAIMS

1. A compound of formula (I):

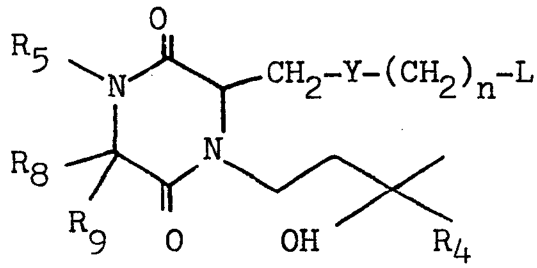

(I)

characterised in that
n is 1 to 5;
L is $CO_2R$ wherein R is hydrogen or $CO_2R$ is an ester group in which R contains from 1 to 12 carbon atoms, or $CH_2COR_1$ wherein $R_1$ is $C_{1-4}$ alkyl;
Y is $-CH_2CH_2-$, $-CH{=}CH-$ or $-C{\equiv}C-$;
$R_2$ is hydrogen or $C_{1-4}$ alkyl;
$R_4$ is hydrogen or $C_{1-9}$ alkyl, $C_{3-8}$ cycloalkyl, $C_{3-8}$ cycloalkyl-$C_{1-6}$ alkyl; or
$R_2$ and $R_4$ taken with the carbon atom to which they are joined represent a $C_{5-8}$ cycloalkyl group;
$R_5$ is $NR_6R_7$ wherein $R_6$ and $R_7$ independently are hydrogen, $C_{1-4}$ alkyl, phenyl $C_{1-4}$ alkyl or $NR_6R_7$ is a 3 to 7 membered heterocyclic group containing only one hetero atom; hydrogen, $C_{1-4}$ alkyl or $C_{1-4}$ alkyl substituted by an OH, $C_{1-4}$ alkoxy, CN, halogen or $NR_6R_7$ group as defined above, or by one or two $CO_2A$ groups in which A is hydrogen or a group containing from 1 to 12 carbon atoms; and $R_8$ and $R_9$ independently are hydrogen, $C_{1-4}$ alkyl or together with the carbon atom to which they are joined are a $C_{3-6}$ cycloalkyl group; and salts thereof.

2. A compound according to claim 1 of formula (III):

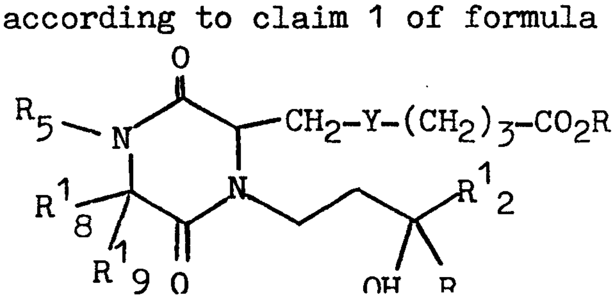

(III)

characterised in that
Y, R, $R_4$ and $R_5$ are as defined in claim 1;
$R^1_2$ is hydrogen, methyl or ethyl;
$R^1_8$ and $R^1_9$ are the same and are hydrogen, $C_{1-4}$ alkyl or together with the carbon atom to which they are joined are a $C_{3-6}$ cycloalkyl group;
and salts thereof.

3. A compound according to claim 1 of formula (IV):

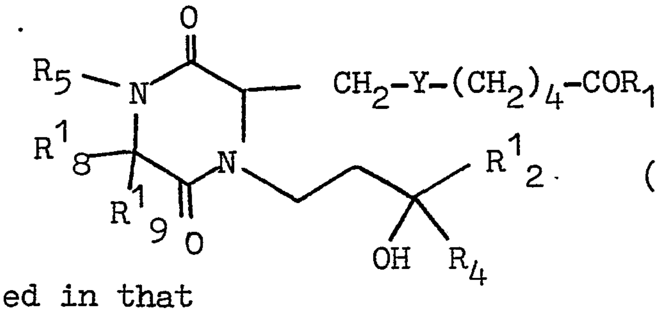

(IV)

characterised in that
$R_1$ is $C_{1-4}$ alkyl and the remaining variables are as defined in claim 2; and salts thereof.

4. A compound according to claim 1 of formula (V) and salts thereof:

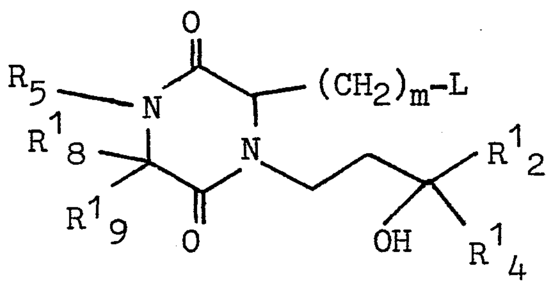

(V)

characterised in that
m is 5, 6, 7 or 8;
$R_4^1$ is hydrogen or $C_{1-9}$ alkyl;
L and $R_5$ are as defined in claim 1; and the remaining variables are as defined in claim 2.

5. 1-(3'-hydroxy-3'-methylnonyl)-4-methyl-6-(6"-methoxycarbonylhexyl)piperazine-2,5-dione.

6. A compound according to claim 1 of formula (VI):

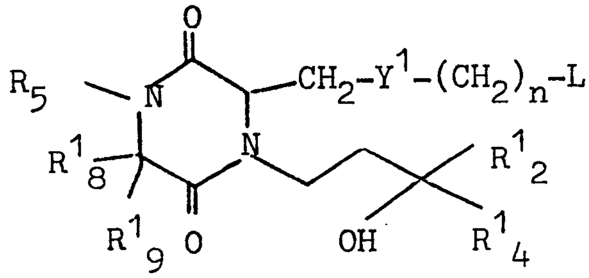

(VI)

characterised in that
$Y^1$ is -CH=CH- or -C≡C-;
n, L and $R_5$ are as defined in claim 1;
$R^1_4$ is as defined in claim 4 , and the remaining variables are as defined in claim 2 and salts thereof.

7. A compound according to claim 1 of formula (VII):

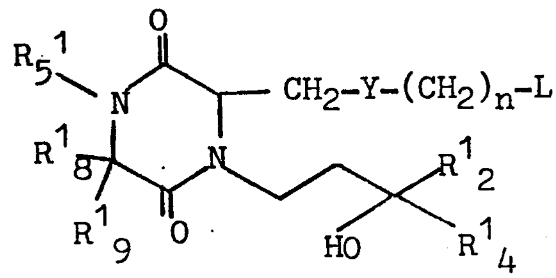

(VII)

charaterised in that $R_5^1$ is hydrogen or $C_{1-4}$ alkyl, and the remaining variables are as defined in claim 4, and salts thereof.

8. A compound according to claim 1 of formula (XI):

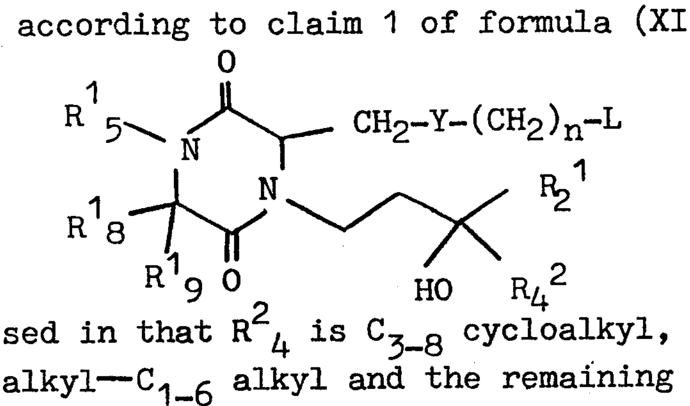

(XI)

characterised in that $R^2_4$ is $C_{3-8}$ cycloalkyl, or $C_{3-8}$ cycloalkyl—$C_{1-6}$ alkyl and the remaining variables are as defined in claim 7, and salts thereof.

9. A compound according to claim 8 of formula (XII):

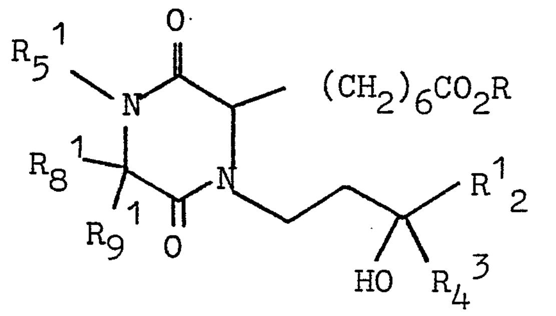

(XII)

characterised in that $R^{3}_{4}$ is a group of formula (II):

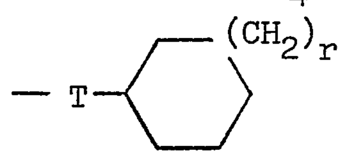

(II)

wherein r is 0 to 3; and
T is a bond or $C_{1-6}$ alkylene and the remaining variables are as defined in claim 8, and salts thereof.

10. 1-(3'-hydroxy-3'-cyclohexylbutyl)-4-methyl-6-(6"-methoxycarbonylhexyl)piperazine-2,5-dione.

11. 1-(3'-hydroxy-3'-cyclohexylbutyl)-4-methyl-6-(6"-carboxyhexyl)piperazine-2,5-dione.

12. The sodium salt of the compound of claim 11.

13. A pharmaceutical composition comprising a compound according to claim 1 together with a pharmaceutically acceptable carrier.

14. A pharmaceutical composition comprising the compound of any one of claims 10 to 12 together with a pharmaceutically acceptable carrier.

15. A process for the preparation of a compound of formula (I), characterised by cyclising a compound of formula (VIII):

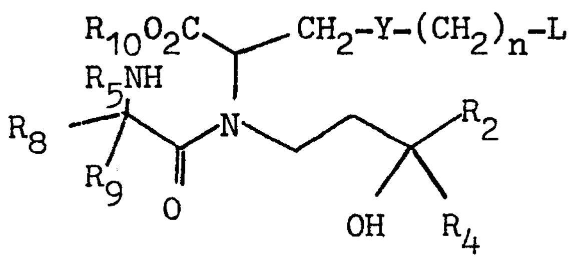

(VIII)

wherein:

$R_{10}$ is hydrogen or $CO_2R_{10}$ is an ester group in which $R_{10}$ contains from 1 to 12 carbon atoms; and the remaining variables are as previously defined; with elimination of $R_{10}OH$; and thereafter as desired converting R, $R_5$, $R_6$, $R_7$ and/or Y in the compound of formula (I) thus formed into other R, $R_5$, $R_6$, $R_7$ or Y.

16. A process according to claim 13 characterised in that the compound of formula (VIII) is formed _in situ_ by reacting a compound of formula (IX):

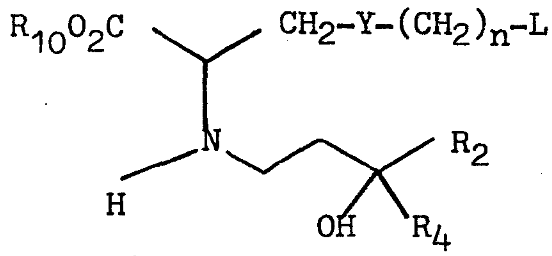

(IX)

wherein the variables are as previously defined, with a compound of formula (X):

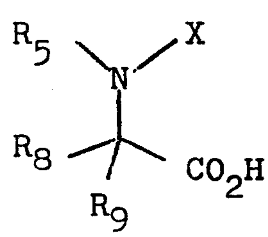

(X)

wherein:

X is a conventional amine protecting group; and the remaining variables are as previously defined; or with such a compound containing a reactive derivative of the $CO_2H$ group thereof; and therafter replacing X conventionally by a hydrogen atom.

17. 1-(3'-hydroxy-3',4'-dimethyloctyl)-4-methyl-6-(6"-methoxycarbonylhexyl)piperazine-2,5-dione.

European Patent Office

# EUROPEAN SEARCH REPORT

0008186

Application number

EP 79 30 1518

| **DOCUMENTS CONSIDERED TO BE RELEVANT** | | | CLASSIFICATION OF THE APPLICATION (Int. Cl.[3]) |
|---|---|---|---|
| Category | Citation of document with indication, where appropriate, of relevant passages | Relevant to claim | |
| A | BE - A - 861 956 (BEECHAM)<br>----- | | C 07 D 241/08<br>A 61 K 31/495 //<br>A 61 K 31/557 |
| | | | TECHNICAL FIELDS SEARCHED (Int.Cl.[3]) |
| | | | C 07 D 241/08<br>A 61 K 31/495 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| √ | The present search report has been drawn up for all claims | | &: member of the same patent family, corresponding document |

| Place of search | Date of completion of the search | Examiner |
|---|---|---|
| The Hague | 24-09-1979 | BERTE |

EPO Form 1503.1 06.78